(19) Europäisches Patentamt
European Patent Office
Office européen des brevets

(11) **EP 4 615 423 B1**

(12) **EUROPEAN PATENT SPECIFICATION**

(45) Date of publication and mention of the grant of the patent:
**02.09.2026 Bulletin 2026/36**

(21) Application number: **23801435.1**

(22) Date of filing: **06.11.2023**

(51) International Patent Classification (IPC):
***A61K 9/20*** *(2006.01)* ***A61K 9/28*** *(2006.01)*
***A61K 31/155*** *(2006.01)*

(52) Cooperative Patent Classification (CPC):
**A61K 9/2846; A61K 9/2009; A61K 9/2054; A61K 9/2072; A61K 9/2095; A61K 9/2866; A61K 31/155**

(86) International application number:
**PCT/EP2023/080846**

(87) International publication number:
**WO 2024/099971 (16.05.2024 Gazette 2024/20)**

(54) **METFORMIN MINITABLETS**

METFORMIN-MINITABLETTEN

MINI-COMPRIMÉS DE METFORMINE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **07.11.2022 EP 22205906**

(43) Date of publication of application:
**17.09.2025 Bulletin 2025/38**

(73) Proprietor: **Rejuvenate Biomed NV**
**3590 Diepenbeek (BE)**

(72) Inventors:
- **BELIËN, Ann**
  **3590 Diepenbeek (NL)**
- **CORVELEYN, Sam**
  **3590 Diepenbeek (BE)**
- **VERHOEVEN, Ellen**
  **9052 Gent (BE)**

(74) Representative: **Arnold & Siedsma**
**Bezuidenhoutseweg 57**
**2594 AC The Hague (NL)**

(56) References cited:
**US-A1- 2006 222 709** **US-A1- 2009 142 378**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 615 423 B1

## Description

FIELD OF THE INVENTION

The present invention relates to a method for obtaining prolonged release metformin minitablets, the minitablets obtainable by the method, and the use of these minitablets as a mono-therapeutic, or in combination with other active pharmaceuticals.

BACKGROUND TO THE INVENTION

Aging is the gradual loss of function and deterioration at the cellular, tissue, and organ level, leading to a progressive loss of physiological integrity, an increased susceptibility to disease and external stressors, and eventually leading to death. As the global population aging is increasing, the incidences of age-related diseases are expanding every year. And hence, numerous attempts have been made in trying to treat the age-related diseases, as well as trying to delay the onset of the complex process of aging. As a result, a number of age-related pathways have been identified that might be targeted to extend life span and health span. For example, there is overwhelming evidence that single gene mutations in nutrient-sensing pathways, such as insulin/insulin-like growth factor (IGF) signalling or the mechanistic target of rapamycin (mTOR) signalling pathways, extend life span and health span in invertebrates. These pathways have also been evaluated in mammalian models, in which health span and life span have been extended by genetic manipulation or drugs. Although this raises hope for new interventions, including drugs that slow the aging process and slow the appearance of age-related disease by modulating conserved pathways of aging, so far, except for some symptomatic treatment, unfortunately, there is no known intervention that was shown to efficiently slow down the human aging process. After all, in addition to treating existing diseases and disorders by means of medicine, the necessity and demand for measures for staying healthy and delaying aging is increasing.

Metformin has widely been used and is approved as an anti-diabetic drug for the treatment of type 2 diabetes. It increases insulin sensitivity, and thereby improves insulin action at the cellular level without affecting insulin secretion. It has also been shown that metformin exerts positive effects on several cardiovascular risk factors. Furthermore, it has been shown that metformin targets a number of aging mechanisms as well. Specifically for aging, metformin leads to decreased insulin levels, decreased IGF-1 signalling, inhibition of mTOR, inhibition of mitochondrial complex I in the electron transport chain and reduction of endogenous production of reactive oxygen species, activation of AMP-activated kinase (AMPK), and reduction in DNA damage. Metformin was also shown to favourably influence metabolic and cellular processes closely associated with the development of age-related conditions, such as inflammation, autophagy, and cellular senescence (Barzilai et al., Cell Metab. 2016). Using a *C. elegans* model system, the health-promoting and life-prolonging effects of metformin in type 2 diabetes were confirmed as well. Human studies have further shown that metformin significantly reduces the risk of cancer in diabetic patients (Fuming et al. Oncol Lett. 2018) and lowers the risk for coronary disease (Hong et al., Diabetes Care. 2014). However, all these effects have been observed when administering metformin at a considerably high therapeutic dose, which is at least 850 mg/day or more. In addition, so far, no synergistic effects of metformin in combination with another compound on age-related diseases were identified.

Galantamine, an acetylcholinesterase inhibitor that allosterically modulates nicotinic receptors, is widely known as a drug administered to patients with Alzheimer's disease. In *C. elegans,* galantamine was shown to facilitate cholinergic neurotransmission in a similar manner as in humans, and to rescue the paralysis phenotype in a transgenic *C. elegans* Alzheimer's disease model (Xin et al., Plos One, 2013), but no effects on locomotion, mobility or other forms of age-related decline have been described for galantamine in *C. elegans.* In humans, it has been shown that galantamine significantly reduces death by myocardial infarction (Nordström et al., 2013). Furthermore, galantamine alleviates inflammation and insulin resistance in metabolic syndrome subjects (Consolim-Colombo et al.; JCI Insight. 2017). However, all these effects have been observed when administering galantamine in a considerably high therapeutic dose, which is at least 24 mg/day or more. Furthermore, also for galantamine, no synergistic effects on age-related diseases were identified when galantamine is combined with another compound.

Earlier work of the applicant (EP3813882A1) has shown a potentiating and even a synergistic effect on age-related diseases using the biguanide metformin, in combination with the acetylcholinesterase inhibitor galantamine. In particular, this effect was even observed when administering at least one of the compounds, or both compounds, in their subtherapeutic dose.

To overcome therapeutic obstacles such as impaired swallowing and polypharmacy therapy, and also offering some therapeutic benefits such as dose flexibility and combined release patterns, minitablets are a promising patient-friendly drug delivery system (Aleksovski et al. Expert Opinion on Drug Delivery 2014 12, 65). Minitablets are tablets typically with a diameter ≤ 3 mm produced on conventional tablet presses equipped with multiple tooling. The production of minitablets is similar to the production of standard tablets but requires excellent powder flow due to the small dies, exact control of process parameters and special caution during tablet press assembly in order to avoid tool damage.

US2009142378 discloses prolonged release tablets of metformin in which the blend with PVA solution takes place in a fluid bed to make granules. Also in US2006222709 sustained release metformin tablets were made by fluid bed granulation of the active ingredient with filler and binder. In contrast, in the present invention, no fluid bed blending of the ingredients is used, but instead dry powder blending was used, followed by direct compression of the blend. This method was surprisingly found to result in particular advantages especially in terms of powder flowability and low friability of the compressed cores.

It is therefore an object of the current invention to address problems associated with the production of minitablets, by providing a novel method for the production of metformin minitablets, the minitablets obtainable by the method, as well the use of metformin minitablets as a mono-therapeutic, or in combination with other therapeutics, in the treatment of age-related diseases.

## SUMMARY OF THE INVENTION

According to a first aspect, the present invention provides a method for the preparation of prolonged release minitablets for oral administration comprising metformin or a pharmaceutical salt thereof as active pharmaceutical ingredient, said method comprising the following steps: (1) blending the active pharmaceutical ingredient with a first glidant and a first binder, thereby generating a first blend; (2) blending the first blend obtained in step (1) with a filler, a second glidant and a lubricant, thereby obtaining a second blend; (3) tableting the second blend obtained in step (2), thereby forming minitablets; preparing a coating suspension; and (5) coating of the minitablets obtained in step (3) with the coating suspension of step (4).

In particular, the present invention provides a method for the preparation of prolonged release minitablets for oral administration comprising metformin or a pharmaceutical salt thereof as active pharmaceutical ingredient, said method comprising the following steps: (1) blending the active pharmaceutical ingredient with a first glidant and a first binder, thereby generating a first dry powder blend; (2) blending the first dry powder blend obtained in step (1) with a filler, a second glidant and a lubricant, thereby obtaining a second dry powder blend; (3) tableting the second dry powder blend obtained in step (2) by direct compression, thereby forming minitablets; preparing a coating suspension; and (5) coating of the minitablets obtained in step (3) with the coating suspension of step (4).

According to an embodiment of the invention, preparing said coating suspension comprises the following steps: (4a) preparing a first dispersion comprising a second binder; (4b) blending the first suspension obtained in step (4a) with a surfactant, an anti-tacking agent, and a sustained release polymer, thereby generating said coating suspension; and (4c) optionally filter the coating suspension obtained in step (4b).

According to a particular embodiment of the invention, said coating suspension is filtered through a 500 $\mu$m mesh screen.

According to an embodiment of the invention, coating of the minitablets occurs via fluidized air bed coating or perforated pan coating. According to a particular embodiment of the invention, coating of the minitablets occurs via fluidized air bed coating with bottom spray of the coating suspension.

According to different embodiments of the invention, said minitablets obtained in step (5) have a coating mass, relative to the total mass of the coated minitablet, in the range of 5 to 40 wt%, preferably in the range of 10 to 35 wt%, more preferably in the range of 15 to 30 wt%.

According to different embodiments of the invention, said minitablets obtained in step (3) have a mean diameter in the range of 1 to 3 mm, preferably in the range of 1.5 to 2.5 mm, more preferably in the range of 1.8 to 2.2 mm.

According to different embodiments of the invention, said minitablets comprise 15-35 % w/w active pharmaceutical ingredient, 0.1-1 % w/w first glidant; 0.1-1 % w/w second glidant; 1-5 % w/w first binder; 1-5 % w/w second binder; 35-60 % w/w filler; 0.5-5 % w/w lubricant; 0.5-5 % w/w surfactant; 5-20 % w/w sustained release polymer and 5-20 % w/w anti-tacking agent.

According to different embodiments of the invention, said first binder is selected from the list comprising: hydroxypropylmethylcellulose, acacia gum, alginic acid, carbomer, carboxymethylcellulose calcium, carboxymethycellulose sodium, microcrystalline cellulose, powdered cellulose, ethyl cellulose, gelatine, liquid glucose, guar gum, hydroxyethyl cellulose, hydroxypropyl cellulose, low substituted hydroxypropyl cellulose, hydroxypropylmethyl cellulose (hypromellose), magnesium aluminum silicate, maltodextrin, methylcellulose, polydextrose, polyethylene oxide, povidone, copovidone, sodium alginate, starch paste, pregelatinized starch, sucrose (syrup) and mixtures thereof. According to particular embodiments, said first binder is hydroxypropylcellulose.

According to different embodiments of the invention, said second binder is selected from the list comprising: hydroxypropylmethylcellulose, acacia gum, alginic acid, carbomer, carboxymethylcellulose calcium, carboxymethycellulose sodium, microcrystalline cellulose, powdered cellulose, ethyl cellulose, gelatine, liquid glucose, guar gum, hydroxyethyl cellulose, hydroxypropyl cellulose, low substituted hydroxypropyl cellulose, hydroxypropylmethyl cellulose (hypromellose), magnesium aluminum silicate, maltodextrin, methylcellulose, polydextrose, polyethylene oxide, povidone, copovidone, sodium alginate, starch paste, pregelatinized starch, sucrose (syrup) and mixtures thereof. According

to particular embodiments, said second binder is hydroxypropylcellulose.

According to different embodiments of the invention, said filler is selected from the list comprising: microcrystalline cellulose, calcium carbonate, calcium phosphate (dibasic), calcium phosphate (tribasic), calcium sulphate, cellulose, microcrystalline cellulose, microcrystalline silicified cellulose, powdered cellulose, dextrates, dextrose, fructose, lactitol, lactose monohydrate, magnesium carbonate, maltitol, maltodextrin, maltose, mannitol, sodium chloride, sorbitol, starch, pregelatinized starch, sucrose, compressible sugar, xylitol. According to particular embodiments, said first filler is microcrystalline cellulose.

According to different embodiments of the invention, said first glidant is selected from the list comprising: colloidal hydrated silica, colloidal silica, corn starch, talc. According to particular embodiments, said first glidant is colloidal hydrated silica.

According to different embodiments of the invention, said second glidant is selected from the list comprising: colloidal hydrated silica, colloidal silica, corn starch, talc. According to particular embodiments, said second glidant is colloidal hydrated silica.

According to different embodiments of the invention, said lubricant is selected from the list comprising: magnesium stearate, sodium stearyl fumarate, calcium stearate, fumaric acid, glyceryl behenate, glyceryl palmitostearate, hydrogenated vegetable oil, magnesium lauryl sulphate, magnesium stearate, sodium lauryl sulphate, sodium stearyl fumarate, starch, stearic acid, talc, zinc stearate and mixtures thereof. According to particular embodiments, said lubricant is sodium stearyl fumarate.

According to different embodiments of the invention, said surfactant is selected from polysorbate, sodium dodecyl sulphate, sodium lauryl sulphate. According to particular embodiments, said surfactant is polysorbate.

According to different embodiments of the invention, said sustained release polymer is selected from polymers of methacrylic acid or methacrylic acid and ethyl acrylate, and cellulosic polymers such as cellulose acetate phthalate (CAP), hydroxypropyl methyl cellulose phthalate (HPMCP), hydroxypropyl methyl cellulose acetate succinate (HPMCAS) and carboxymethyl ethyl cellulose (CMEC). According to particular embodiments of the invention, said sustained release polymer is a polymer resulting from a mixture of methacrylic acid and ethyl acrylate monomers.

According to different embodiments of the invention, said anti-tacking agent is selected from talc, magnesium stearate. According to particular embodiments, said anti-tacking agent is talc.

According to a further aspect, the present invention provides a minitablet obtainable by said method.

According to yet a further aspect, the present invention provides a prolonged release minitablet for oral administration, obtainable by the methods as disclosed herein, and comprising metformin or a pharmaceutical salt thereof as an active pharmaceutical ingredient; wherein the uncoated minitablet has a mean diameter in the range of 1 to 3 mm, preferably in the range of 1.5 to 2.5 mm, more preferably in the range of 1.8 to 2.2 mm;

wherein the minitablet is coated with a sustained release polymer; wherein the coating mass, relative to the total mass of the coated minitablet, in the range of 5 to 40 wt%, preferably in the range of 10 to 35 wt%, more preferably in the range of 15 to 30 wt%.

According to yet a further aspect, the present invention provides a pharmaceutical composition comprising a minitablet according to embodiments of the invention.

## BRIEF DESCRIPTION OF THE DRAWINGS

**FIG. 1A** shows the dissolution profile of examples 1a, 1b, 1c, and 1d, as shown in Table 6, in a phosphate buffer at a pH of 6.8.

**FIG. 1B** shows the dissolution profile of examples 1a, 1b, 1c, and 1d, as shown in Table 6, in 0.1 M HCl solution at a pH of 1.

**FIG. 2** shows the dissolution profile of example 1d, as shown in Table 6, and comparative examples 2, and 4 in a phosphate buffer at a pH of 6.8.

## DETAILED DESCRIPTION OF THE INVENTION

The present invention will now be further described. In the following paragraphs, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

As used in the specification and the appended claims, the singular forms "a", "an", and "the" include plural referents unless the context clearly dictates otherwise. By way of example, "a compound" means one compound or more than one compound. The terms described above and others used in the specification are well understood to those in the

art. The compounds of the present invention can be prepared according to the reaction scheme provided in the examples hereinafter, but those skilled in the art will appreciate that these are only illustrative for the invention and that the compounds of this invention can be prepared by any of several standard synthetic processes commonly used by those skilled in the art of organic chemistry.

According to a first aspect, the present invention provides a method for the preparation of prolonged release minitablets for oral administration comprising metformin or a pharmaceutical salt thereof as active pharmaceutical ingredient, said method comprising the following steps: (1) blending the active pharmaceutical ingredient with a first glidant and a first binder, thereby generating a first blend; (2) blending the first blend obtained in step (1) with a filler, a second glidant and a lubricant, thereby obtaining a second blend; (3) tableting the second blend obtained in step (2), thereby forming minitablets; preparing a coating suspension; and (5) coating of the minitablets obtained in step (3) with the coating suspension of step (4).

In particular, the present invention provides a method for the preparation of prolonged release minitablets for oral administration comprising metformin or a pharmaceutical salt thereof as active pharmaceutical ingredient, said method comprising the following steps: (1) blending the active pharmaceutical ingredient with a first glidant and a first binder, thereby generating a first dry powder blend; (2) blending the first dry powder blend obtained in step (1) with a filler, a second glidant and a lubricant, thereby obtaining a second dry powder blend; (3) tableting the second dry powder blend obtained in step (2) by direct compression, thereby forming minitablets; preparing a coating suspension; and (5) coating of the minitablets obtained in step (3) with the coating suspension of step (4).

The terms 'prolonged release' and 'sustained release' are used synonymously for a drug release extending over a prolonged period of time, in contrast to 'immediate release', where a drug is released immediately after administration. According to Ph. Eur. 2.9.3, sustained or prolonged release shows a drug release where 1) no unit is above 50% release after 90 min, and 2) no unit is above 80% release after 180 min.

The term 'dry powder' as used herein refers to a formulation of ingredients that is in a powdered form and does not contain a liquid component, such as water. In the present invention the first and second blends as used herein are obtained by blending or mixing a set of dry powder ingredients in order to obtain a dry powder blend. The blends of the invention are not obtained using fluid-based techniques such as fluid bed granulation or wet bed granulation, and do not require the use of fluids in order to obtain such blends. Accordingly, the method of the present invention is characterized in that it does not include a step of fluid or wet bed granulation.

In the context of the present invention, the term 'direct compression' is meant to be the compression of the dry blend directly into the minitablet form, without the need for wet granulation or other intermediate steps. In particular, the dry powder blend is fed directly into a tablet press, where it is compressed into minitablets of the desired size and shape.

According to an embodiment of the invention, preparing said coating suspension comprises the following steps: (4a) preparing a first dispersion comprising a second binder; (4b) blending the first suspension obtained in step (4a) with a surfactant, an anti-tacking agent, and a sustained release polymer, thereby generating said coating suspension; and (4c) optionally filter the coating suspension obtained in step (4b).

According to a further particular embodiment, said coating suspension may comprise additional ingredients, for example to improve visual appearance and/or taste of the coated minitablets. Examples of such additional ingredients may be colorants, flavoring agents, sweeteners, glazing agents, anti-tacking agents,....

According to a particular embodiment of the invention, said coating suspension is filtered through a 500 $\mu$m mesh screen.

According to an embodiment of the invention, coating of the minitablets occurs via fluidized air bed coating or perforated pan coating. According to a particular embodiment of the invention, coating of the minitablets occurs via fluidized air bed coating with bottom spray of the coating suspension.

According to different embodiments of the invention, said minitablets obtained in step (5) have a coating concentration (mass), relative to the total mass of the coated minitablet, in the range of 5 to 40 wt%, preferably in the range of 10 to 35 wt%, more preferably in the range of 15 to 30 wt%.

It should be appreciated that minitablets obtained in step (5) refer to coated minitablets, according to the different embodiments of the invention.

According to different embodiments of the invention, said minitablets obtained in step (3) a mean diameter in the range of 1 to 3 mm, preferably in the range of 1.5 to 2.5 mm, more preferably in the range of 1.8 to 2.2 mm.

It should be appreciated that minitablets obtained in step (3) refer to uncoated minitablets, according to the different embodiments of the invention.

According to different embodiments of the invention, said minitablets comprise 15-35 % w/w active pharmaceutical ingredient, 0.1-1 % w/w first glidant; 0.1-1 % w/w second glidant; 1-5 % w/w first binder; 1-5 % w/w second binder; 35-60 % w/w filler; 0.5-5 % w/w lubricant; 0.5-5 % w/w surfactant; 5-20 % w/w sustained release polymer and 5-20 % w/w anti-tacking agent.

According to different embodiments of the invention, no more than 50% of the metformin or a pharmaceutical salt thereof of said minitablets is released within the first 90 minutes.

According to different embodiments of the invention, no more than 80% of the metformin or a pharmaceutical salt thereof of said minitablets is released within the first 90 minutes.

According to different embodiments of the invention, said first binder is selected from the list comprising: hydroxypropylmethylcellulose, acacia gum, alginic acid, carbomer, carboxymethylcellulose calcium, carboxymethycellulose sodium, microcrystalline cellulose, powdered cellulose, ethyl cellulose, gelatine, liquid glucose, guar gum, hydroxyethyl cellulose, hydroxypropyl cellulose, low substituted hydroxypropyl cellulose, hydroxypropylmethyl cellulose (hypromellose), magnesium aluminum silicate, maltodextrin, methylcellulose, polydextrose, polyethylene oxide, povidone, copovidone, sodium alginate, starch paste, pregelatinized starch, sucrose (syrup) and mixtures thereof. According to particular embodiments, said first binder is hydroxypropylcellulose.

According to different embodiments of the invention, said second binder is selected from the list comprising: hydroxypropylmethylcellulose, acacia gum, alginic acid, carbomer, carboxymethylcellulose calcium, carboxymethycellulose sodium, microcrystalline cellulose, powdered cellulose, ethyl cellulose, gelatine, liquid glucose, guar gum, hydroxyethyl cellulose, hydroxypropyl cellulose, low substituted hydroxypropyl cellulose, hydroxypropylmethyl cellulose (hypromellose), magnesium aluminum silicate, maltodextrin, methylcellulose, polydextrose, polyethylene oxide, povidone, copovidone, sodium alginate, starch paste, pregelatinized starch, sucrose (syrup) and mixtures thereof. According to particular embodiments, said second binder is hydroxypropylcellulose.

According to different embodiments of the invention, said filler is selected from the list comprising: microcrystalline cellulose, calcium carbonate, calcium phosphate (dibasic), calcium phosphate (tribasic), calcium sulphate, cellulose, microcrystalline cellulose, microcrystalline silicified cellulose, powdered cellulose, dextrates, dextrose, fructose, lactitol, lactose monohydrate, magnesium carbonate, maltitol, maltodextrin, maltose, mannitol, sodium chloride, sorbitol, starch, pregelatinized starch, sucrose, compressible sugar, xylitol. According to particular embodiments, said first filler is microcrystalline cellulose.

According to different embodiments of the invention, said first glidant is selected from the list comprising: colloidal hydrated silica, colloidal silica, corn starch, talc. According to particular embodiments, said first glidant is colloidal hydrated silica.

According to different embodiments of the invention, said second glidant is selected from the list comprising: colloidal hydrated silica, colloidal silica, corn starch, talc. According to particular embodiments, said second glidant is colloidal hydrated silica.

According to different embodiments of the invention, said lubricant is selected from the list comprising: magnesium stearate, sodium stearyl fumarate, calcium stearate, fumaric acid, glyceryl behenate, glyceryl palmitostearate, hydrogenated vegetable oil, magnesium lauryl sulphate, magnesium stearate, sodium lauryl sulphate, sodium stearyl fumarate, starch, stearic acid, talc, zinc stearate and mixtures thereof. According to particular embodiments, said lubricant is sodium stearyl fumarate.

According to different embodiments of the invention, said surfactant is selected from polysorbate, sodium dodecyl sulphate, sodium lauryl sulphate. According to particular embodiments, said surfactant is polysorbate.

According to different embodiments of the invention, said sustained release polymer is selected from polymers of methacrylic acid or methacrylic acid and ethyl acrylate, and cellulosic polymers such as cellulose acetate phthalate (CAP), hydroxypropyl methyl cellulose phthalate (HPMCP), hydroxypropyl methyl cellulose acetate succinate (HPMCAS) and carboxymethyl ethyl cellulose (CMEC). According to particular embodiments of the invention, said sustained release polymer is a polymer resulting from a mixture of methacrylic acid and ethyl acrylate monomers.

According to different embodiments of the invention, said anti-tacking agent is selected from talc, and magnesium stearate. According to particular embodiments of the invention, said anti-tacking agent is talc.

According to particular embodiments of the invention, said minitablets comprise metformin or a pharmaceutical salt thereof, hydroxypropylcellulose, microcrystalline cellulose, colloidal hydrated silica, sodium stearyl fumarate, polysorbate, polymethacrylate and talc.

According to particular embodiments of the invention, said minitablets comprise 15-35 % w/w metformin, 0.2-2 % w/w colloidal hydrated silica; 2-10 % w/w hydroxypropylcellulose; 35-60 % w/w microcrystalline cellulose; 0.5-5 % w/w sodium stearyl fumarate; 0.5-5 % w/w polysorbate; 5-20 % w/w polymethacrylate and 5-20 % w/w talc.

According to a further aspect, the present invention provides a minitablet obtainable by said method.

According to yet a further aspect, the present invention provides a prolonged release minitablet for oral administration comprising metformin or a pharmaceutical salt thereof as an active pharmaceutical ingredient;

wherein the uncoated minitablet has a mean diameter in the range of 1 to 3 mm, preferably in the range of 1.5 to 2.5 mm, more preferably in the range of 1.8 to 2.2 mm;

wherein the minitablet is coated with a sustained release polymer; wherein the coating mass, relative to the total mass of the coated minitablet, in the range of 5 to 40 wt%, preferably in the range of 10 to 35 wt%, more preferably in the range of 15 to 30 wt%.

According to yet a further aspect, the present invention provides a pharmaceutical composition comprising a minitablet according to embodiments of the invention. Such pharmaceutical compositions can be prepared and formulated by methods known in the art and may take a wide variety of forms depending on the form of preparation desired for administration. These pharmaceutical compositions are desirably in unitary dosage form suitable, preferably, for systemic administration such as oral, percutaneous, or parenteral administration.

For example, the minitablets can be formulated along with common excipients, diluents, or carriers, and formed into oral tablets, capsules, sprays, mouth washes, oral liquids (e.g. suspensions, solutions, emulsions), powders, or any other suitable dosage form.

According to an embodiment of the invention, said pharmaceutical composition can be used on its own or as a pharmaceutical combination with another agent used for prevention, stabilization and/or reduction of age-related complaints, degenerative dysfunctioning and/or degenerative complaints. According to an embodiment of the invention, said pharmaceutical composition can be used on its own or as a pharmaceutical combination with another agent for improving a measure of life span and/or health span. According to a particular embodiment, said pharmaceutical composition comprises as another agent a acetylcholinesterase inhibitor, and/or a hydrate, a pharmaceutically acceptable salt or solvate thereof. According to a more particular embodiment, said pharmaceutical composition comprises as another agent galantamine.

## EXAMPLES

### Materials

Metformin.HCl (Met.HCl) was purchased from Fagron. Microcrystalline cellulose (MCC - Avicel® PH102) was purchased from FMC Health and Nutrition. Magnesium Stearate (Ligamed MF-2-V) and Talcum (talc) were purchased from was purchased from IMCD Benelux. Sodium Stearyl fumarate (Pruv) was purchased from JRS Pharma. Colloidal hydrated silica (Syloid 244FP) was purchased from Grace Davison. Hydroxypropylmethylcellulose (HPMC E5) was purchased from Colorcon. Ethylcellulose (Aqualon EC-N10) and Hydroxypropylcellulose (Klucel EXF ULTRA Pharm) were purchased from Ashland. Poly(ethyl acrylate-co-methyl methacrylate) 2:1 (Eudragit NM30D) was purchased from Evonik. Polysorbate 80 (Tween 80) was purchased from VWR.

### Methods

*Bulk and tapped density*

The bulk and tapped density of the powders were determined using a Tap Density Tester TD1 (Sotax, Allschwil, Switzerland) equipped with a 25 mL graduated cylinder (readable to 0.5 mL). The tap height and tap speed were set at 3 mm and 250 taps/min. Approximately 25 mL of material was poured into a 25 mL graduated cylinder. The powder weight and exact volume were used to calculate the bulk density (ρB). Subsequently, the sample was subjected to 10, 500, and 1250 taps and the corresponding volumes V10, V500, and V1250 were determined to the nearest graduated unit. In case the difference between V500 and V1250 was less than or equal to 2 mL, V1250 was retained as the tapped volume. When the difference between the volume after 500 taps differed more than 2 mL from the volume obtained after 1250 taps, 1250 extra taps were conducted. The volume reading was then used to determine the tapped density (ρT). Finally, the hausner ratio (HR) and compressibility index (CI) were calculated and used as a measure of powder flowability:

$$Hausner\ ratio = \frac{\rho T}{\rho B}$$

$$Compressibility\ index = 100\ x\ \frac{(\rho T - \rho B)}{\rho T}$$

*Dimensions*

Immediately after tableting, the diameter and height of the minitablets (n = 20 per batch) were recorded using a digital caliper (Mahr, Göttingen, Germany).

*Weight*

The individual weight of 30 minitablets (of each batch) was recorded using an analytical 5d balance (Sartorius

ME235P, Göttingen, Germany).

*Hardness testing*

The diametral breaking force of the minitablets (n = 6 per batch) was measured using a pharmaceutical tablet hardness tester (Sotax HT10, Basel, Switzerland).

*Disintegration testing*

A Ph. Eur. disintegration apparatus (Sotax DT2, Basel, Switzerland) was used to determine the disintegration time of the minitablets (n = 3 per batch). Considering the small diameter of minitablets, screen opening dimensions were reduced to 1.4 x 1.4 mm. All tests were performed in elixTM water at a temperature of 37 $\pm$ 0.5 °C using disks.

*Friability measurements*

Minitablet friability was determined by subjecting approximately 6.5 g of minitablets (in accordance with Ph. Eur. Standards) using a friabilator apparatus (Sotax FT2, Basel, Switzerland), set at a speed of 25 rpm for 4 minutes. The percentage weight loss was expressed as the tablet friability.

*Assay testing*

Metformin was quantified by Ultra High-Performance Liquid Chromatography - UPLC (Ph Eur 2.2.29), using UV-absorbance measurement at 230 nm. The evaluation is based on peak-area measurement and external standardization with relative response.

**Example 1 - Sustained release (reservoir system)**

*Stage 1 - Pre-blend*

A pre-blend was prepared by weighing (and sieving/calibrating on 600 μm) Syloid 244FP (about 11 g), HPC Klucel (about 119 g) and Met.HCl (about 1210 g), followed by blending for about 30 min using a Turbula T2A blender.

*Stage 2 - Blend*

A blend was prepared by weighing (and sieving/calibrating on 600 μm) Avicel PH102 (about 2568 g), Pre-blend (about 1341 g), Syloid 244FP (about 11 g) and Pruv (about 80 g), subsequently introducing them into a suitable container in the following order: ½ Avicel PH102, Syloid 244FP, Pre-blend, Pruv, ½ Avicel PH102, and blending for about 20 min using a Turbula T2A blender to obtain a white homogeneous blend.

Composition data of the obtained blend is provided in Table 1.

***Table 1. Composition data of Pre-Blend & Blend according to Example 1***

| Pre-Blend | | | | | |
|---|---|---|---|---|---|
| | **Compound** | **Mass total batch (g)** | **Mass (mean) per minitablet (mg)** | **Relative (%)** |
| | Colloidal silica (Syloïd 244FP) | 11.35 | 0.02 | 0.3% |
| | HPC (Klucel EXF ULTRA Pharm) | 119.35 | 0.21 | 3.0% |
| | Met.HCl | 1210 | 2.16 | 30.3% |
| | **Subtotal** | **1340.7** | **2.39** | **33.5%** |

| Blend | | | | |
|---|---|---|---|---|
| | **Compound** | **Mass total batch (g)** | **Mass (mean) per minitablet (mg)** | **Relative (%)** |
| | MCC (Avicel PH 102) | 2568 | 4.58 | 64.2% |
| | Colloidal silica (Syloïd 244FP) | 11.35 | 0.02 | 0.3% |
| | Sodium Stearyl fumarate (Pruv) | 79.55 | 0.14 | 2.0% |
| | **Subtotal** | **2658.9** | **4.75** | **66.5%** |
| **Total Blend** | | **3999.6** | **7.14** | **100.0%** |

Analysis data of the obtained blend is provided in Table 2.

***Table 2. Analysis data of Blend according to Example 1***

| | |
|---|---|
| Bulk density (g/ml) | 0.36 |
| Tapped density (g/ml) | 0.47 |
| Compressibility index (%) | 22 |
| Hausner ratio | 1.28 |
| **Aptitude to flowability** | **passable** |
| **Aptitude to compressibility** | **intermediate compressibility** |
| LOD (%) | 3.36 |

*Stage 3 - Tableting*

The tableting step was performed on an eccentric tablet press (Korsch XP1, Korsch AG, Berlin, Germany) equipped with 8 D2 mm punches. Tableting parameters: mean compression force: 5.5-6.5 kN.

Analysis data of the obtained minitablets is provided in Table 3.

***Table 3. Analysis data of Minitablets according to Example 1***

| | |
|---|---|
| Appearance | White round brightening tablets |
| Friability (n=20) | 0.325 % |
| Hardness (n=6, mean ± SD) | 12 ± 1 N |
| Height (n=20, mean ± SD) | 1.98 ± 0.03 mm |
| Mass (n=20, mean ± SD) | 7.14 ± 0.12 mg |

Observations: The resulting minitablets possess a high abrasion resistance. Hence the friability of the minitablets is well below 1.0 (Ph. Eur. standard for tablets) and 0.5% (guideline for minitablets intended for coating). In addition to a low friability value, the height of the minitablets is similar to the diameter (i.e. 2.0 mm) of the tablets, which results in an aspect ratio of approximately 1 (beneficial for coating).

*Stage 4 - Coating suspension*

A coating suspension was prepared by:

1) adding HMPC E5 (about 28 g) to purified water and stirring for about 30 min using an overhead stirrer;

2) adding polysorbate 80 (about 28 g) to purified water and stirring for about 75 min at increasing stirring speed;

3) adding the polysorbate solution to the HMPC solution under overhead stirring for about 10 min;

4) adding talc (about 273 g) stepwise under vigorous stirring, and stirring further (after incorporation) for about 10 min; and

5) adding the solution to Eudragit NM30D (about 244 g) and stirring further for 5 min.

Optionally the coating suspension can be filtered through a 500 μm screen.
Composition data of the obtained coating suspension is provided in Table 4.

***Table 4. Coating composition (dry weight) according to Example 1***

| **Coating** | | | |
|---|---|---|---|
| | **Compound** | **Mass (g)** | **Relative (%)** |
| | Methacrylic copolymer (Eudragit NM30D) | 244.3 | 42.6 |
| | HPMC (Methocel E5 Premium LV) | 28.4 | 4.9 |
| | Polysorbate 80 (Montanox 80 PHA Premium) | 28.4 | 4.9 |
| | Talc | 272.75 | 47.5 |
| | **Subtotal** | **573.85** | **100.0** |

*Stage 5 - Coating*

Minitablets (immediate release) as described in Stage 3 were introduced into a suitable product container of the fluid bed equipment (Oystar Hüttlin Mycrolab). Coating was performed with the suspension prepared in the previous stage according to the parameters listed in Table 5 and further curing for about 24 h at about 40 °C, to obtain dull white coated minitablets.

***Table 5. Coating parameters***

| **Granulation parameters** | **Inlet flow rate ($m^3$/h)** | **Inlet temperature (°C)** | **Product temperature (°C)** | **Outlet temperature (°C)** | **Duration (min)** |
|---|---|---|---|---|---|
| Pre-heating | ± 150 | ± 45 | 40-45 | 25-40 | 5-10 |
| Coating | 85-135 | ± 50 | 25-45 | 25-45 | 125-155 |
| Drying | ± 130 | ± 45 | ± 40 | 35-40 | ± 10 |

During the coating process, coated minitablet samples were collected, and additional coating liquid was sprayed onto the minitablets during the coating process. This resulted in different minitablet samples (Examples 1a (control), 1b, 1c, and 1d) with different increasing weight and coating thicknesses/weights, as shown in Table 6.

***Table 6. Coating weights for minitablets according to Example 1***

| **Example** | **Mass (mean) (mg)** | **Coating mass (mean) (mg)** | **Weight increase (w/w) (%)** | **Relative coating amount (wt%)** |
|---|---|---|---|---|
| 1a (control) | 7.14 | 0 | 0 | 0 |
| 1b | 7.59 | 0.43 | 5.9 | 5.6 |
| 1c | 8.70 | 1.54 | 17.9 | 15.2 |
| 1d | 9.58 | 2.42 | 25.5 | 20.3 |

*Stage 6 - Dissolution testing*

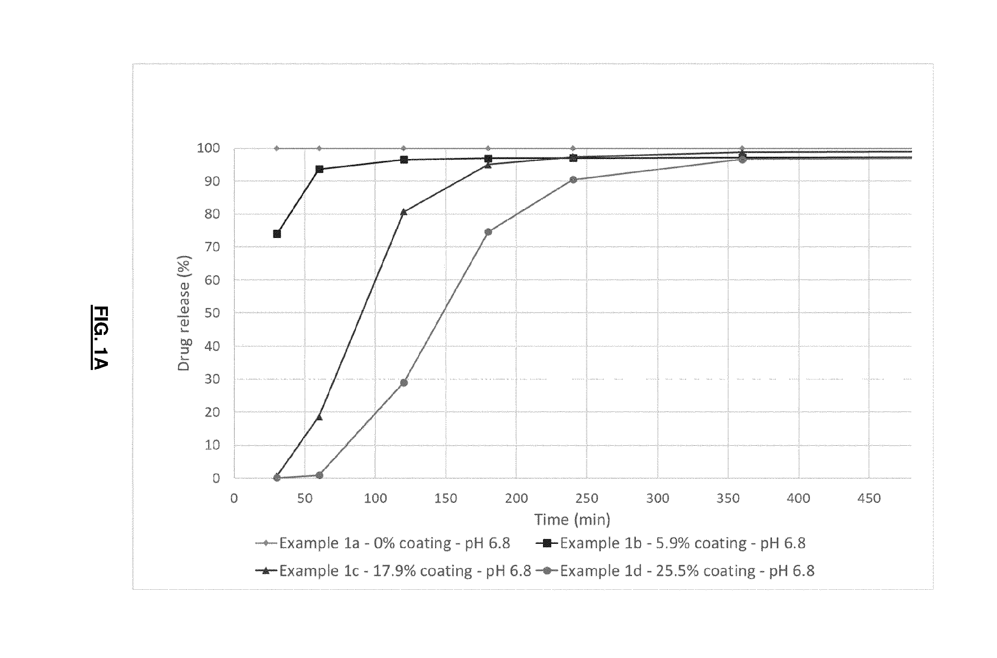

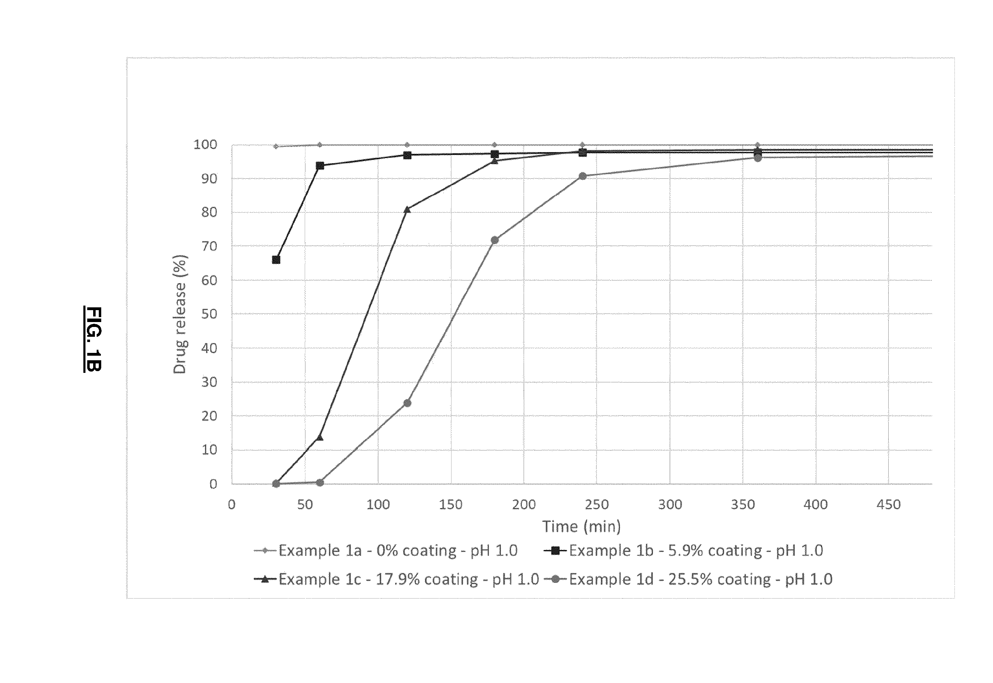

The (coated) minitablets were filled into capsules (with a dose strength of eq. 100 mg) and subjected to in vitro dissolution testing using two different media (pH 1.0 and pH 6.8).
Observations: As shown in Figure 1A and 1B, slower release kinetics were observed with increasing coating weight. Furthermore, it was found that medium pH did not affect metformin release from coated minitablets.

**Comparative example 2 - Sustained release (matrix system)**

*Stage 1 - Pre-blend*

A pre-blend was prepared by weighing (and sieving/calibrating on 600 μm) Syloid 244FP (about 3 g), and Met.HCl (about 605 g), followed by blending for about 30 min using a Turbula T2A blender.

*Stage 2 - Blend*

A blend was prepared by weighing (and sieving/calibrating on 600 μm) Avicel PH102 (about 15 g), Pre-blend of Stage 1 (about 12 g), HPMC (Metolose 90 SH 100,000 SR) (about 12 g) and Ligamed MF-2V (about 0.8 g), subsequently introducing them into a suitable container and blending for about 20 min using a Turbula T2A blender to obtain a white homogeneous blend.

Composition data of the obtained blend is provided in Table 7.

***Table 7. Composition data of Pre-Blend & Blend according to Comparative example 2***

| **Pre-Blend** | | | | |
|---|---|---|---|---|
| **Compound** | | **Mass total batch (g)** | **Mass (mean) per minitablet (mg)** | **Relative (%)** |
| Colloidal silica (Syloïd 244FP) | | 2.85 | - | 0.5% |
| Met.HCl | | 605 | - | 99.5% |
| **Subtotal** | | **607.85** | - | **100.0%** |
| **Blend** | | | | |
| **Compound** | | **Mass total batch (g)** | **Mass (mean) per minitablet (mg)** | **Relative (%)** |
| Pre-Blend | | 12 | 1.67 | 30.0% |
| | Colloidal silica (Syloïd 244FP) | 0.06 | 0.01 | 0.14% |
| | Met.HCl | 11.94 | 1.66 | 29.9% |
| MCC (Avicel PH 102) | | 15.2 | 2.11 | 38.0% |
| HPMC (Metolose 90 SH 100.000 SR) | | 12 | 1.67 | 30.0% |
| Magnesium stearate (Ligamed MF-2V) | | 0.8 | 0.11 | 2.0% |
| **Total Blend** | | **40.00** | **5.55** | **100.0%** |

Analysis data of the obtained blend is provided in Table 8.

***Table 8. Analysis data of Blend of Comparative example 2***

| | |
|---|---|
| Hausner ratio | 1.35 |

*Stage 3 - Tableting*

The tableting step was performed on an eccentric tablet press (Korsch XP1, Korsch AG, Berlin, Germany) equipped with 8 D2 mm punches. Tableting parameters: mean compression force: 5.5-6.5 kN.

Observations: In contrast to the blends used for the manufacturing of immediate release metformin HCl minitablets, poor flow properties and capping phenomena were observed at a tableting speed of 15 strokes/min.

*Stage 4 - Dissolution testing*

The HPMC 90 SH 100.000 SR minitablets were filled into capsules (with a dose strength of eq. 100 mg) and tested during in vitro dissolution experiments.

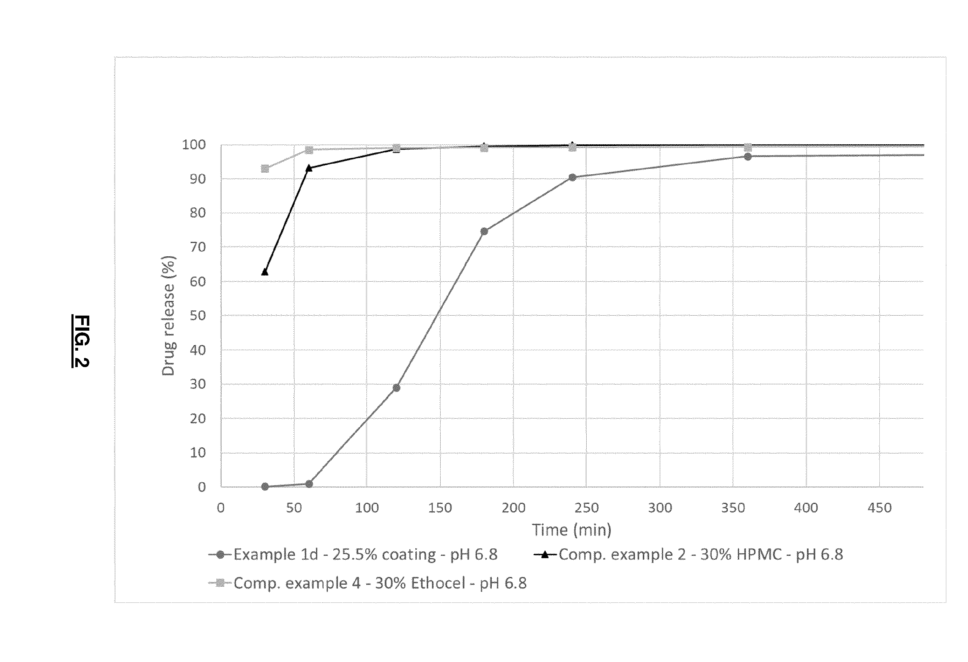

Observations: As shown in Figure 2, the use of 30% (w/w) HPMC 90 SH 100.000 SR did not result in sustained release kinetics, compared to Example 1d, as shown in Table 6.

**Comparative example 3 - Sustained release (matrix system)**

*Stage 2 - Blend*

A blend was prepared by weighing (and sieving/calibrating on 600 μm) Avicel PH102 (about 18 g), Pre-blend of Comparative example 1 (about 30 g), HPMC (Metolose 90 SH 100,000 SR) (about 50 g) and Ligamed MF-2V (about 2 g), subsequently introducing them into a suitable container and blending for about 20 min using a Turbula T2A blender to obtain a white homogeneous blend.

Composition data of the obtained blend is provided in Table 9.

***Table 9. Composition data of Pre-Blend & Blend according to Comparative example 2***

| **Pre-Blend** | | | | | |
|---|---|---|---|---|---|
| | **Compound** | | **Mass total batch (g)** | **Mass (mean) per minitablet (mg)** | **Relative (%)** |
| | Colloidal silica (Syloïd 244FP) | | 2.85 | - | 0.5% |
| | Met.HCl | | 605 | - | 99.5% |
| | **Subtotal** | | **607.85** | - | **100.0%** |
| **Blend** | | | | | |
| | **Compound** | | **Mass total batch (g)** | **Mass (mean) per minitablet (mg)** | **Relative (%)** |
| | Pre-Blend | | 30 | 1.67 | 30.0% |
| | | Colloidal silica (Syloïd 244FP) | 0.14 | 0.01 | 0.14% |
| | | Met.HCl | 29.86 | 1.66 | 29.9% |
| | MCC (Avicel PH 102) | | 18 | 1.00 | 18.0% |
| | HPMC (Metolose 90 SH 100.000 SR) | | 50 | 2.78 | 50.0% |
| | Magnesium stearate (Ligamed MF-2V) | | 2 | 0.11 | 2.0% |
| **Total Blend** | | | **100.00** | **5.55** | **100.0%** |

Analysis data of the obtained blend is provided in Table 10.

***Table 10. Analysis data of Blend of Comparative example 3***

| | |
|---|---|
| Hausner ratio | 1.41 |

*Stage 3 - Tableting*

The tableting step was performed on an eccentric tablet press (Korsch XP1, Korsch AG, Berlin, Germany) equipped with 8 D2 mm punches. Tableting parameters: mean compression force: 5.5-6.5 kN.

Observations: Poor powder flow properties of the blend were observed during tableting, resulting in a non-homogeneous filling of the die. Therefore, it was concluded that it was no viable option to increase the HPMC 90SH-100.000 SR concentration.

**Comparative example 4 - Sustained release (matrix system)**

*Stage 2 - Blend*

A blend was prepared by weighing (and sieving/calibrating on 600 μm) Avicel PH102 (about 30 g), Pre-blend of Comparative example 2 (about 30 g), Ethylcellulose (Ethocel) (about 30 g) and Ligamed MF-2V (about 2 g), subsequently introducing them into a suitable container and blending for about 20 min using a Turbula T2A blender to obtain a white homogeneous blend.

Composition data of the obtained blend is provided in Table 11.

***Table 11. Composition data of Pre-Blend & Blend according to Comparative example 4***

| **Pre-Blend** | | | | | |
|---|---|---|---|---|---|
| | **Compound** | | **Mass total batch (g)** | **Mass (mean) per minitablet (mg)** | **Relative (%)** |
| | Colloidal silica (Syloïd 244FP) | | 2.85 | - | 0.5% |
| | Met.HCl | | 605 | - | 99.5% |
| | **Subtotal** | | **607.85** | - | **100.0%** |
| **Blend** | | | | | |
| | **Compound** | | **Mass total batch (g)** | **Mass (mean) per minitablet (mg)** | **Relative (%)** |
| | Pre-Blend | | 30 | 2.10 | 30.0% |
| | | Colloidal silica (Syloïd 244FP) | 0.14 | 0.01 | 0.14% |
| | | Met.HCl | 29.86 | 2.09 | 29.9% |
| | MCC (Avicel PH 102) | | 38 | 2.66 | 30.0% |
| | Ethylcellulose (Ethocel) | | 30 | 2.10 | 30.0% |
| | Magnesium stearate (Ligamed MF-2V) | | 2 | 0.14 | 2.0% |
| **Total Blend** | | | **100.00** | **7.00** | **100.0%** |

Analysis data of the obtained blend is provided in Table 12.

***Table 12. Analysis data of Blend of Comparative example 4***

| | |
|---|---|
| Hausner ratio | 1.20 |

*Stage 3 - Tableting*

The tableting step was performed on an eccentric tablet press (Korsch XP1, Korsch AG, Berlin, Germany) equipped with 8 D2 mm punches. Tableting parameters: mean compression force: 5.5-6.5 kN.

Observations: Better flow properties and more homogenous die filling during tableting trials compared to HPMC as a matrix forming agent. Friability values of the resulting minitablets were higher than for HPMC based minitablets.

*Stage 4 - Dissolution testing*

The ethylcellulose minitablets were filled into capsules (with a dose strength of eq. 100 mg) and tested during in vitro dissolution experiments.

Observations: As shown in Figure 2, the use of 30% (w/w) ethylcellulose did not result in sustained release kinetics, compared to Example 1d, as shown in Table 6. A similar release pattern was observed when using 50% (w/w) ethylcellulose.

## Claims

**1.** A method for the preparation of prolonged release minitablets for oral administration comprising metformin or a pharmaceutical salt thereof as an active pharmaceutical ingredient, the process comprising the following steps:

(1) blending the active pharmaceutical ingredient with a first glidant and a first binder, thereby generating a first dry powder blend;
(2) blending the first blend obtained in step (1) with a filler, a second glidant and a lubricant, thereby obtaining a second dry powder blend;
(3) tableting the second blend obtained in step (2) by direct compression, thereby forming minitablets;
(4) preparing a coating suspension; and
(5) coating of the minitablets obtained in step (3) with the coating suspension of step (4).

2. The method according to claim 1 wherein preparing the coating suspension of step (4) comprises the following steps:

(4a) preparing a first dispersion comprising a second binder;
(4b) blending the first suspension obtained in step (4a) with a surfactant, an anti-tacking agent, and a sustained release polymer, thereby generating said coating suspension; and
(4c) optionally filter the coating suspension obtained in step (4b).

3. The method according to any one of claims 1 to 2, wherein coating of the minitablets of step (5) is done using a fluidized bed system or a perforated pan coating system, in particular the coating is done using a fluidized bed system with bottom spray of the coating suspension.

4. The method according to any one of claims 1 to 3, wherein the coated minitablets have a coating mass, relative to the total mass of the coated minitablet, in the range of 5 to 40 wt%, preferably in the range of 10 to 35 wt%, more preferably in the range of 15 to 30 wt%.

5. The method according to any one of claims 1 to 4, wherein the uncoated minitablets have a mean diameter in the range of 1 to 3 mm, preferably in the range of 1.5 to 2.5 mm, more preferably in the range of 1.8 to 2.2 mm.

6. The method according to any one of claims 1 to 5, wherein the minitablets comprise 15-35 % w/w of metformin or a pharmaceutical salt thereof as an active pharmaceutical ingredient, 0.1-1 % w/w first glidant; 0.1-1 % w/w second glidant; 1-5 % w/w first binder; 1-5 % w/w second binder; 35-60 % w/w filler; 0.5-5 % w/w lubricant; 0.5-5 % w/w surfactant; 5-20 % w/w sustained release polymer and 5-20 % w/w anti-tacking agent.

7. The method according to any one of claims 1 to 6, wherein the glidant is selected from colloidal hydrated silica, colloidal silica, corn starch, talc; preferably wherein the glidant is colloidal hydrated silica.

8. The method according to any one of claims 1 to 7, wherein the lubricant is selected from the list comprising: magnesium stearate, sodium stearyl fumarate, calcium stearate, fumaric acid, glyceryl behenate, glyceryl palmitostearate, hydrogenated vegetable oil, magnesium lauryl sulphate, magnesium stearate, sodium lauryl sulphate, sodium stearyl fumarate, starch, stearic acid, talc, zinc stearate and mixtures thereof; preferably wherein the lubricant is sodium stearyl fumarate.

9. The method according to any one of claims 1 to 8, wherein the surfactant is selected from polysorbate, sodium dodecyl sulphate, sodium lauryl sulphate; preferably wherein the surfactant is polysorbate.

10. The method according to any one of claims 1 to 9, wherein the sustained release polymer is selected from polymers of methacrylic acid or methacrylic acid and ethyl acrylate, and cellulosic polymers such as cellulose acetate phthalate (CAP), hydroxypropyl methyl cellulose phthalate (HPMCP), hydroxypropyl methyl cellulose acetate succinate (HPMCAS) and carboxymethyl ethyl cellulose (CMEC), preferably wherein the sustained release polymer is a polymer resulting from a mixture of methacrylic acid and ethyl acrylate monomers.

11. The method according to any one of claims 1 to 10, wherein the anti-tacking agent is selected from talc, magnesium stearate; preferably wherein the anti-tacking agent is talc.

12. A minitablet obtainable by the method according to any one of claims 1 to 11.

13. A minitablet as defined in claim 12;

wherein the uncoated minitablet has a mean diameter in the range of 1 to 3 mm, preferably in the range of 1.5 to 2.5 mm, more preferably in the range of 1.8 to 2.2 mm; and
wherein the minitablet is coated with a sustained release polymer; wherein the coating mass, relative to the total mass of the coated minitablet, in the range of 5 to 40 wt%, preferably in the range of 10 to 35 wt%, more preferably in the range of 15 to 30 wt%.

14. The minitablet according to claim 13, wherein the sustained release polymer is selected from polymers of methacrylic acid or methacrylic acid and ethyl acrylate, and cellulosic polymers such as cellulose acetate phthalate (CAP), hydroxypropyl methyl cellulose phthalate (HPMCP), hydroxypropyl methyl cellulose acetate succinate (HPMCAS) and carboxymethyl ethyl cellulose (CMEC), preferably wherein the sustained release polymer is a polymer resulting

from a mixture of methacrylic acid and ethyl acrylate monomers.

**15.** A pharmaceutical composition comprising the minitablet according to any one of claims 12 to 14.

**Patentansprüche**

**1.** Verfahren für die Herstellung von Minitabletten mit verlängerter Freisetzung für eine orale Verabreichung, umfassend Metformin oder ein pharmazeutisches Salz davon als pharmazeutischen Wirkstoff, der Prozess umfassend die folgenden Schritte:

(1) Mischen des pharmazeutischen Wirkstoffs mit einem ersten Fließregulierungsmittel und einem ersten Bindemittel, wobei dadurch eine erste Trockenpulvermischung erzeugt wird;
(2) Mischen der in Schritt (1) erhaltenen ersten Mischung mit einem Füllstoff, einem zweiten Fließregulierungsmittel und einem Gleitmittel, wobei dadurch eine zweite Trockenpulvermischung erhalten wird;
(3) Tablettieren der in Schritt (2) erhaltenen zweiten Mischung durch Direktverpressung, wobei dadurch Minitabletten ausgebildet werden;
(4) Herstellen einer Beschichtungssuspension; und
(5) Beschichten der in Schritt (3) erhaltenen Minitabletten mit der Beschichtungssuspension von Schritt (4).

**2.** Verfahren nach Anspruch 1, wobei das Herstellen der Beschichtungssuspension von Schritt (4) die folgenden Schritte umfasst:

(4a) Herstellen einer ersten Dispersion, umfassend ein zweites Bindemittel;
(4b) Mischen der in Schritt (4a) erhaltenen ersten Suspension mit einem Tensid, einem Antihaftmittel und einem Polymer mit verzögerter Freisetzung, wobei dadurch die Beschichtungssuspension erzeugt wird; und
(4c) optional Filtern der in Schritt (4b) erhaltenen Beschichtungssuspension.

**3.** Verfahren nach einem der Ansprüche 1 bis 2, wobei das Beschichten der Minitabletten von Schritt (5) unter Verwendung eines Wirbelschichtsystems oder eines Beschichtungssystems mit perforierter Wanne erfolgt, insbesondere die Beschichtung unter Verwendung eines Wirbelschichtsystems mit Bodensprühung der Beschichtungssuspension erfolgt.

**4.** Verfahren nach einem der Ansprüche 1 bis 3, wobei die beschichteten Minitabletten eine Beschichtungsmasse, relativ zu der Gesamtmasse der beschichteten Minitablette, in dem Bereich von 5 bis 40 Gew.-%, vorzugsweise in dem Bereich von 10 bis 35 Gew.-%, mehr bevorzugt in dem Bereich von 15 bis 30 Gew.-%, aufweisen.

**5.** Verfahren nach einem der Ansprüche 1 bis 4, wobei die unbeschichteten Minitabletten einen mittleren Durchmesser in dem Bereich von 1 bis 3 mm, vorzugsweise in dem Bereich von 1,5 bis 2,5 mm, mehr bevorzugt in dem Bereich von 1,8 bis 2,2 mm, aufweisen.

**6.** Verfahren nach einem der Ansprüche 1 bis 5, wobei die Minitabletten zu 15-35 Gew.-% Metformin oder ein pharmazeutisches Salz davon als pharmazeutischen Wirkstoff, zu 0,1-1 Gew.-% das erste Fließregulierungsmittel; zu 0,1-1 Gew.-% das zweite Fließregulierungsmittel; zu 1-5 Gew.-% das erste Bindemittel; zu 1-5 Gew.-% das zweite Bindemittel; zu 35-60 Gew.-% den Füllstoff; zu 0,5-5 Gew.-% das Gleitmittel; zu 0,5-5 Gew.-% das Tensid; zu 5-20 Gew.-% das Polymer mit verzögerter Freisetzung und zu 5-20 Gew.-% das Antihaftmittel umfassen.

**7.** Verfahren nach einem der Ansprüche 1 bis 6, wobei das Fließregulierungsmittel aus kolloidalem hydratisiertem Siliciumdioxid, kolloidalem Siliciumdioxid, Maisstärke, Talkum ausgewählt ist; wobei das Fließregulierungsmittel vorzugsweise kolloidales hydratisiertes Siliciumdioxid ist.

**8.** Verfahren nach einem der Ansprüche 1 bis 7, wobei das Gleitmittel aus der Liste ausgewählt ist, umfassend: Magnesiumstearat, Natriumstearylfumarat, Calciumstearat, Fumarsäure, Glycerylbehenat, Glycerylpalmitostearat, hydriertes Pflanzenöl, Magnesiumlaurylsulfat, Natriumlaurylsulfat, Stärke, Stearinsäure, Talkum, Zinkstearat und Gemische davon; wobei das Gleitmittel vorzugsweise Natriumstearylfumarat ist.

**9.** Verfahren nach einem der Ansprüche 1 bis 8, wobei das Tensid aus Polysorbat, Natriumdodecylsulfat, Natriumlaurylsulfat ausgewählt ist; wobei das Tensid vorzugsweise Polysorbat ist.

**10.** Verfahren nach einem der Ansprüche 1 bis 9, wobei das Polymer mit verzögerter Freisetzung aus Polymeren von Methacrylsäure oder Methacrylsäure und Ethylacrylat, und cellulosischen Polymeren wie Celluloseacetatphthalat (CAP), Hydroxypropylmethylcellulosephthalat (HPMCP), Hydroxypropylmethylcelluloseacetatsuccinat (HPMCAS) und Carboxymethylethylcellulose (CMEC) ausgewählt ist, wobei das Polymer mit verzögerter Freisetzung vorzugsweise ein Polymer ist, das aus einem Gemisch von Methacrylsäure- und Ethylacrylatmonomeren resultiert.

**11.** Verfahren nach einem der Ansprüche 1 bis 10, wobei das Antihaftmittel aus Talkum, Magnesiumstearat ausgewählt ist; wobei das Antihaftmittel vorzugsweise Talkum ist.

**12.** Minitablette, die durch das Verfahren nach einem der Ansprüche 1 bis 11 erhältlich ist.

**13.** Minitablette, wie in Anspruch 12 definiert;

wobei die unbeschichtete Minitablette einen mittleren Durchmesser in dem Bereich von 1 bis 3 mm, vorzugsweise in dem Bereich von 1,5 bis 2,5 mm, mehr bevorzugt in dem Bereich von 1,8 bis 2,2 mm, aufweist; und
wobei die Minitablette mit einem Polymer mit verzögerter Freisetzung beschichtet ist; wobei die Beschichtungsmasse, relativ zu der Gesamtmasse der beschichteten Minitablette, in dem Bereich von 5 bis 40 Gew.-%, vorzugsweise in dem Bereich von 10 bis 35 Gew.-%, mehr bevorzugt in dem Bereich von 15 bis 30 Gew.-%, liegt.

**14.** Minitablette nach Anspruch 13, wobei das Polymer mit verzögerter Freisetzung aus Polymeren von Methacrylsäure oder Methacrylsäure und Ethylacrylat und cellulosischen Polymeren wie Celluloseacetatphthalat (CAP), Hydroxypropylmethylcellulosephthalat (HPMCP), Hydroxypropylmethylcelluloseacetatsuccinat (HPMCAS) und Carboxymethylethylcellulose (CMEC) ausgewählt ist, wobei das Polymer mit verzögerter Freisetzung vorzugsweise ein Polymer ist, das aus einem Gemisch von Methacrylsäure- und Ethylacrylatmonomeren resultiert.

**15.** Pharmazeutische Zusammensetzung, umfassend die Minitablette nach einem der Ansprüche 12 bis 14.

## Revendications

**1.** Procédé pour la préparation de minicomprimés à libération prolongée pour administration orale comprenant de la metformine ou un sel pharmaceutique de celle-ci en tant qu'ingrédient pharmaceutique actif, le procédé comprenant les étapes suivantes :

(1) mélange de l'ingrédient pharmaceutique actif avec un premier agent de glissement et un premier liant, permettant ainsi de générer un premier mélange pulvérulent sec ;
(2) mélange du premier mélange obtenu à l'étape (1) avec une charge, un second agent de glissement et un lubrifiant, permettant ainsi d'obtenir un second mélange pulvérulent sec ;
(3) compression du second mélange obtenu à l'étape (2) par compression directe, permettant ainsi de former des minicomprimés ;
(4) préparation d'une suspension d'enrobage ; et
(5) enrobage des minicomprimés obtenus à l'étape (3) avec la suspension d'enrobage de l'étape (4).

**2.** Procédé selon la revendication 1, dans lequel la préparation de la suspension d'enrobage de l'étape (4) comprend les étapes suivantes :

(4a) préparation d'une première dispersion comprenant un second liant ;
(4b) mélange de la première suspension obtenue à l'étape (4a) avec un agent tensioactif, un agent anti-collant et un polymère à libération prolongée, permettant ainsi de générer ladite suspension d'enrobage ; et
(4c) filtration facultative de la suspension d'enrobage obtenue à l'étape (4b).

**3.** Procédé selon l'une quelconque des revendications 1 à 2, dans lequel l'enrobage des minicomprimés de l'étape (5) est effectué à l'aide d'un système à lit fluidisé ou d'un système d'enrobage à plateau perforé, en particulier l'enrobage est effectué à l'aide d'un système à lit fluidisé avec pulvérisation inférieure de la suspension d'enrobage.

**4.** Procédé selon l'une quelconque des revendications 1 à 3, dans lequel les minicomprimés enrobés ont une masse d'enrobage, par rapport à la masse totale du minicomprimé enrobé, comprise dans la plage de 5 à 40 % en poids, de préférence dans la plage de 10 à 35 % en poids, plus préférablement dans la plage de 15 à 30 % en poids.

5. Procédé selon l'une quelconque des revendications 1 à 4, dans lequel les minicomprimés non enrobés ont un diamètre moyen compris dans la plage de 1 à 3 mm, de préférence dans la plage de 1,5 à 2,5 mm, plus préférablement dans la plage de 1,8 à 2,2 mm.

6. Procédé selon l'une quelconque des revendications 1 à 5, dans lequel les minicomprimés comprennent 15 à 35 % p/p de metformine ou d'un sel pharmaceutique de celle-ci en tant qu'ingrédient pharmaceutique actif, 0,1 à 1 % p/p de premier agent de glissement ; 0,1 à 1 % p/p de second agent de glissement ; 1 à 5 % p/p de premier liant ; 1 à 5 % p/p de second liant ; 35 à 60 % p/p de charge ; 0,5 à 5 % p/p de lubrifiant ; 0,5 à 5 % p/p d'agent tensioactif ; 5 à 20 % p/p de polymère à libération prolongée et 5 à 20 % p/p d'agent anti-collant.

7. Procédé selon l'une quelconque des revendications 1 à 6, dans lequel l'agent de glissement est choisi parmi silice hydratée colloïdale, silice colloïdale, amidon de maïs, talc ; de préférence dans lequel l'agent de glissement est la silice hydratée colloïdale.

8. Procédé selon l'une quelconque des revendications 1 à 7, dans lequel le lubrifiant est choisi dans la liste comprenant : stéarate de magnésium, stéarylfumarate de sodium, stéarate de calcium, acide fumarique, béhénate de glycéryle, palmitostéarate de glycéryle, huile végétale hydrogénée, laurylsulfate de magnésium, laurylsulfate de sodium, amidon, acide stéarique, talc, stéarate de zinc et mélanges de ceux-ci ; de préférence dans lequel le lubrifiant est le stéarylfumarate de sodium.

9. Procédé selon l'une quelconque des revendications 1 à 8, dans lequel l'agent tensioactif est choisi parmi polysorbate, dodécylsulfate de sodium, laurylsulfate de sodium ; de préférence dans lequel l'agent tensioactif est le polysorbate.

10. Procédé selon l'une quelconque des revendications 1 à 9, dans lequel le polymère à libération prolongée est choisi parmi des polymères d'acide méthacrylique ou d'acide méthacrylique et d'acrylate d'éthyle, et des polymères cellulosiques tels que phtalate-acétate de cellulose (CAP), phtalate d'hydroxypropylméthylcellulose (HPMCP), succinate-acétate d'hydroxypropylméthylcellulose (HPMCAS) et carboxyméthyléthylcellulose (CMEC), de préférence dans lequel le polymère à libération prolongée est un polymère résultant d'un mélange de monomères d'acide méthacrylique et d'acrylate d'éthyle.

11. Procédé selon l'une quelconque des revendications 1 à 10, dans lequel l'agent anti-collant est choisi parmi talc, stéarate de magnésium ; de préférence dans lequel l'agent anti-collant est le talc.

12. Minicomprimé pouvant être obtenu par le procédé selon l'une quelconque des revendications 1 à 11.

13. Minicomprimé selon la revendication 12 ;

dans lequel le minicomprimé non enrobé a un diamètre moyen compris dans la plage de 1 à 3 mm, de préférence dans la plage de 1,5 à 2,5 mm, plus préférablement dans la plage de 1,8 à 2,2 mm ; et
dans lequel le minicomprimé est enrobé d'un polymère à libération prolongée ; dans lequel la masse d'enrobage, par rapport à la masse totale du minicomprimé enrobé, est comprise dans la plage de 5 à 40 % en poids, de préférence dans la plage de 10 à 35 % en poids, plus préférablement dans la plage de 15 à 30 % en poids.

14. Minicomprimé selon la revendication 13, dans lequel le polymère à libération prolongée est choisi parmi des polymères d'acide méthacrylique ou d'acide méthacrylique et d'acrylate d'éthyle, et des polymères cellulosiques tels que phtalate-acétate de cellulose (CAP), phtalate d'hydroxypropylméthylcellulose (HPMCP), succinate-acétate d'hydroxypropylméthylcellulose (HPMCAS) et carboxyméthyléthylcellulose (CMEC), de préférence dans lequel le polymère à libération prolongée est un polymère résultant d'un mélange de monomères d'acide méthacrylique et d'acrylate d'éthyle.

15. Composition pharmaceutique comprenant le minicomprimé selon l'une quelconque des revendications 12 à 14.

Drug release (%)
100
90
80
70
60
50
40
30
20
10
0
0
50
100
150
200
250
300
350
400
450
Time (min)
Example 1a - 0% coating - pH 6.8
Example 1b - 5.9% coating - pH 6.8
Example 1c - 17.9% coating - pH 6.8
Example 1d - 25.5% coating - pH 6.8

**FIG. 1A**

Drug release (%)
100
90
80
70
60
50
40
30
20
10
0
0
50
100
150
200
250
300
350
400
450
Time (min)
Example 1a - 0% coating - pH 1.0
Example 1b - 5.9% coating - pH 1.0
Example 1c - 17.9% coating - pH 1.0
Example 1d - 25.5% coating - pH 1.0

**FIG. 1B**

Drug release (%)
100
90
80
70
60
50
40
30
20
10
0
0
50
100
150
200
250
300
350
400
450
Time (min)
Example 1d - 25.5% coating - pH 6.8
Comp. example 2 - 30% HPMC - pH 6.8
Comp. example 4 - 30% Ethocel - pH 6.8

**FIG. 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3813882 A1
- US 2009142378 A
- US 2006222709 A


### Non-patent literature cited in the description


- **BARZILAI et al.** *Cell Metab.*, 2016
- **FUMING et al.** *Oncol Lett.*, 2018
- **HONG et al.** *Diabetes Care*, 2014
- **XIN et al.** *Plos One*, 2013
- **CONSOLIM-COLOMBO et al.** *JCI Insight*, 2017
- **ALEKSOVSKI et al.** *Expert Opinion on Drug Delivery*, 2014, vol. 12, 65